# EUROPÄISCHE PATENTANMELDUNG

(11) **EP 2 638 951 A1**
(43) Veröffentlichungstag der Anmeldung: **18.09.2013**
(21) Anmeldenummer: 12159508.6
(22) Anmeldetag: 14.03.2012
(51) Int. Cl.: B01D 53/22, B01D 53/75

(54) **Kombinierte Gasaufbereitung**

(71) Anmelder: ARTAN HOLDING AG, 9495 Triesen (LI); Methapower Biogas GmbH, 1010 Wien (AT)
(72) Erfinder: Gruber-Schmidt, Johann, 1180 Wien (AT)
(74) Vertreter: Sonn & Partner Patentanwälte

(57) **Zusammenfassung**

Die vorliegende Erfindung betrifft ein Verfahren zur Entfernung von Kohlendioxid aus einem Kohlendioxid-haltigen Gasgemisch, umfassend eine mehrstufige Gasreinigung, dadurch gekennzeichnet, dass in zumindest einer ersten Stufe das Gasgemisch mit einer Kohlendioxid-absorbierenden Flüssigkeit kontaktiert wird, wodurch ein vorgereinigtes Gasgemisch erhalten wird, und in zumindest einer zweiten Stufe das vorgereinigte Gasgemisch mit einem Kohlendioxid-Adsorber oder einem Molekularsieb kontaktiert wird, wodurch Kohlendioxid vom Gasgemisch getrennt wird sowie eine Vorrichtung zur Durchführung des Verfahrens.

## Beschreibung

Die vorliegende Erfindung betrifft energetisch günstige Verfahren zur Aufbereitung von kohlendioxidhaltigen Gasgemischen, insbesondere von Biogas.

In zahlreichen industriellen und landwirtschaftlichen Verfahren, beispielsweise in Kommunalbetrieben, bei der Verarbeitung von Nahrungsmitteln und Tierfutter und in der Forstwirtschaft wird Biomasse in der Form von Abfall- und Nebenprodukten erzeugt. Die landwirtschaftliche und chemische Industrie sowie öffentliche Betriebe haben ein erhebliches Interesse an der Entwicklung von Verfahren zur Umwandlung dieser Biomassen in Materialien mit einem höheren Nutzwert. So könnten derartige Biomassen beispielsweise unter Verwendung von Mikroorganismen und/oder hydrolytischen Enzymen potentiell in Bioethanol, Biogas oder Chemikalien umgewandelt werden. Allerdings haben die meisten der heutzutage bekannten Verfahren aufgrund ihrer hohen Produktionskosten und der hohen Energieerfordernisse und der infolgedessen innewohnenden unsicheren ökonomischen Realisierbarkeit noch nicht die kommerzielle Anwendung im Großmaßstab erreicht. Es ist daher ein Ziel vieler technologischer Entwicklungen Effizienzsteigerungen zu ermöglichen. Biogas beispielsweise ist ein Gemisch hauptsächlich aus Methan und Kohlendioxid (auch Kohlenstoffdioxid), üblicherweise im Bereich 45% bis 70% Methan und 30% bis 55% Kohlendioxid. Methan ist hierbei das kommerziell wertvolle Produkt, welches entweder vor Ort zur Stromerzeugung oder zur Einspeisung in das Erdgasnetz verwendet wird. Für letztere Anwendung muss allerdings das Rohbiogas aufgereinigt und das Nebenprodukt Kohlendioxid entfernt werden. Die Abreicherung von Kohlendioxid ist ein energieaufwendiger Prozess, welcher die Wirtschaftlichkeit beeinträchtigt.

Die Veröffentlichung DE 10 2005 051 952 beschreibt ein Verfahren zur Herstellung von Methan und flüssigem Kohlendioxid aus Raffinerie- oder Biogas, wobei das ursprüngliche Gasgemisch in einer Absorptionskolonne mit einer Waschflüssigkeit behandelt wird. Kohlendioxid wird hierbei durch die Waschflüssigkeit, welche zur Absorption geeignete Amine enthält, aufgenommen und abgetrennt. Nachteilig ist, dass um ein Produktgas mit hoher Reinheit bzw. hohem Methangehalt zu erhalten, relativ hohe Drücke von bis zu 70 bar eingesetzt werden müssen. Dies macht den Prozess unwirtschaftlich.

In der WO 2008/034473 wird ein ähnliches Verfahren zur Reinigung von Biogas mittels Wäsche beschrieben, wobei Kohlendioxid durch Absorption an eine Waschflüssigkeit aus dem Biogas entfernt wird.

Die DE 10 2008 058 114 beschreibt ein Verfahren zur Reinigung von Rohbiogas wobei in einem oder zwei Schritten, wobei Schwefelwasserstoff, Ammoniak und Kohlendioxid mittels Wasserstoffperoxid und einem Alkalimetallhydroxid abgetrennt werden.

Die US2011/005392 betrifft die Trennung von Kohlendioxid von einem Gas mittels PSA (Druckwechseladsorption - Pressure Swing Adsorption).

Die DE 199 47 339 beschreibt ein Verfahren zur Reinigung von Biogas wobei zur Trennung von Methan und Kohlendioxid mindestens zwei PSA-Trennstufen eingesetzt werden.

In der Praxis werden häufig Verfahren, welche auf einer unterschiedlichen Gaspermeation beruhen, eingesetzt. Ein solches Verfahren ist beispielsweise in der WO 02/26359 für die Trennung von Stickstoff und Sauerstoff beschrieben, kann aber bei Einsatz von geeigneten Membranen und Drücken auch für die Trennung von Kohlendioxid von Methan eingesetzt werden. Derartige Membranen sind beispielsweise in der US 5,674,629 oder der WO 2008/077837 beschrieben. Ein Nachteil der membranbasierten Verfahren ist die geringe Trenneffizienz bei Rohbiogas. In einem Schritt werden nur bis zu 20% des Gases (also bei Biogas weniger als die Hälfte des Kohlendioxidanteils) abgetrennt, weswegen diese Verfahren meist mehrstufig (insb. dreistufig) und bei höheren Ausgangsdrücken von mehr als 12 bar eingesetzt werden. In den ersten beiden Stufen werden jeweils 20% des Gases und in der dritten Stufe 5% des Gases (Kohlendioxid) abgetrennt, wobei Produktgase mit hoher Reinheit (∼97 % - 98 % Methan) erhalten werden. Das abgetrennte Kohlendioxidgas muss letztlich auch entfernt werden können, welches meist durch ein Verflüssigungsverfahren in eine transportable Form überführt wird. Verflüssigungsverfahren sind beispielsweise in der EP 0646756 A1 oder der US 2004/0250682 A1 beschrieben. Zur Verflüssigung sind hohe Drücke und niedrige Temperaturen notwendig, welche die energetische Bilanz des Verfahrens weiters verschlechtert.

In der DE 10 2010 006 649 wurde das Problem der hohen Kosten einer PSA- oder Membran-basierten Reinigung, d.h. Trennung von Kohlendioxid von Methan, von Biogas erkannt. Es wird vorgeschlagen eine dynamische Reinigungsstufe vorzuschalten, in der in einer Zentrifuge Kohlendioxid und Methan aufgrund der unterschiedlichen Massen getrennt werden. Zentrifugen, welche eine sinnvolle hohe Reinheit (80% gemäß der DE 10 2010 006 649) erreichen, sind allerding ebenfalls wartungsintensiv und müssen in mehreren Stufen seriell betrieben werden.

Es ist daher ein Ziel der vorliegenden Erfindung Verfahren zur Trennung von Kohlendioxid aus Gasgemischen zur Verfügung zu stellen, welche wesentlich effizienter und somit günstiger sind.

In einem ersten Aspekt betrifft die Erfindung ein Verfahren zur Entfernung von Kohlendioxid aus einem Kohlendioxid-haltigen Gasgemisch, umfassend eine mehrstufige Gasreinigung, dadurch gekennzeichnet, dass in zumindest einer ersten Stufe das Gasgemisch mit einer Kohlendioxid-absorbierenden Flüssigkeit kontaktiert wird, wodurch ein vorgereinigtes Gasgemisch erhalten wird, und in zumindest einer zweiten Stufe das vorgereinigte Gasgemisch mit einem Kohlendioxid-Adsorber oder einem Molekularsieb kontaktiert wird, wodurch Kohlendioxid vom Gasgemisch getrennt wird. Die Erfindung betrifft kurz eine Verfahren wobei in einer Gaswäsche Kohlendioxid aus einem Gasgemisch aufgenommen wird und anschließend in einer Feinreinigung weiteres Kohlendioxid aus dem Gasgemisch entfernt wird, wobei in Summe eine hohe Reinheit des verbleibenden Gases ermöglicht wird.

Zudem betrifft die Erfindung eine Vorrichtung mit einem Gaswäscher (oder einen Gas-Flüssigkeitskontaktor) mit einer Einleitung für ein Gasgemisch, Ein- und Ausgangsleitungen für eine Waschflüssigkeit, einer Ausgangsleitung für ein vorgereinigtes Gasgemisch, und mit einer Kohlendioxidadsoptionsanlage (z.B. PSA) oder einer Molekularsiebanlage (z.B. Membrananlage) zur Abtrennung von Kohlendioxid aus dem vorgereinigten Gasgemisch. Diese Vorrichtung ist zur Durchführung des erfindungsgemäßen Verfahrens geeignet bzw. hierfür hergerichtet. Weitere Aspekte sind in den Ansprüchen definiert. Die hierin beschriebenen Ausführungen beziehen sich sowohl auf das Verfahren als auch auf die Vorrichtung, für die Mittel zur Durchführung der einzelnen Verfahrensmerkmale vorgesehen werden können. Die Vorrichtung und ihre Teile können im erfindungsgemäßen Verfahren zum Einsatz kommen. Alle Ausführungsformen können miteinander kombiniert werden - selbst in der zweiten Stufe, für welche hierin beispielsweise Alternativen angegeben sind (Adsorption oder Molekularsiebtechnik), lassen sich auch beide Varianten parallel oder infolge seriell einsetzen.

In der erfindungsgemäßen ersten Stufe werden große Mengen Kohlendioxid chemisch absorbiert, vorzugsweise ist die Kapazität zur Aufnahme von mindestens 15% des Gasgemisches vorgesehen. Bei einem Anteil von 45% Kohlendioxid im Gasgemisch (wie z.B. in Biogas) würde also ein Drittel des im Gasgemisch enthaltenen Kohlendioxids absorbiert werden können. Vorzugsweise wird mindestens 18%, speziell bevorzugt mindestens 20%, mindestens 25% oder mindestens 30% des Gasgemisches aufgenommen. Bezogen auf den Kohlendioxidanteil werden mindestens 25%, vorzugsweise mindestens 30%, mindestens 35%, mindestens 40%, mindestens 45%, mindestens 50%, mindestens 55%, mindestens 60%, mindestens 65%, mindestens 70% oder mindestens 75% oder mindestens 80%, des Kohlendioxides des Gasgemisches in dieser ersten Stufe absorbiert. Vorzugsweise ist diese erste Stufe der Kohlendioxidentfernung frei von Wasserstoffperoxid oder anderen oxidierenden Substanzen. Alle %-Angaben verstehen sich im Gasgemisch als Vol.-%. Sofern das Gasgemisch ein methanhaltiges Gas ist, wie Biogas, kann in dieser ersten Stufe der Methananteil auf zwischen 75% bis 92%, vorzugsweise auf zwischen 80 % und 90 % erhöht werden.

Die erste Stufe wird hierin auch als Gaswäsche und die Flüssigkeit der ersten Stufe wird auch als Waschflüssigkeit bezeichnet. Hierbei wird der Gasstrom mit einem Flüssigkeitsstrom in Kontakt gebracht wird, um Kohlendioxid des Gasstroms in der Flüssigkeit aufzunehmen. Methan sollte nicht oder nur in geringen Mengen, z.B. weniger als 5%, vorzugsweise weniger als 1%, des Gasgemisches durch die Flüssigkeit aufgenommen werden. Die Flüssigkeit ist vorzugsweise eine wässrige Flüssigkeit insbesondere Wasser. Die Flüssigkeit kann mindestens 50% oder mindestens 80% Wasser als Lösemittel enthalten.

Durch die Abtrennung durch Waschen liegt Kohlendioxid in flüssiger Form in der Flüssigkeit gebunden vor, ohne das eine aufwändige (kryogene) Verflüssigung vorgenommen werden muss. Das gebundene Kohlendioxid kann daher an einen beliebigen Ort transportiert werden um dort verwertet zu werden. Beispielsweise kann das Kohlendioxid, wie in der WO 2006/006164 beschrieben, verwendet werden um nach Desorption vom Absorptionsmittel (z.B. K₂CO₃, aber auch andere bekannte Substanzen wie Amine oder Basen) der Waschflüssigkeit durch Reaktion mit Wasserstoff Treibstoff (Methan) herzustellen.

Die Flüssigkeit kann beispielsweise Wasser sein, welches vorzugsweise unter hohem Druck (z.B. 8-30 bar), Kohlendioxid aufnimmt. Höhere Absorptionen können durch Kohlendioxid-absorbierende Substanzen erzielt werden, die Kohlendioxid chemisch binden. Vorzugsweise enthält die Flüssigkeit eine solche Kohlendioxid-absorbierende Substanz. Kohlendioxid absorbierende Substanzen sind hinlänglich bekannt und sind beispielsweise Carbonat-, Hydrogencarbonat- oder Bicarbonatbilder, wie beispielsweise Kalk, Dolomit, CaO, Ca(OH)₂, Me₂CO₃, wobei Me ein einwertiges Metallion ist (bildet 2 MeHCO₃ mit Wasser und CO₂), Basen oder Amine, insbesondere Alkyl- oder Alkoholamine, wie beispielsweis in der DE 10 2009 056 661 A1 beschrieben. Vorzugsweise ist Me K oder Na. Geeignete Amine sind beispielsweise MEA (Monoethanolamin), DEA (Diethanolamin), TEA (Triethanolamin), Diethyldiamin, 1,4-Diethylendiamin (Piperazin). Vorzugsweise wird ein Karbonat (vorzugsweise K₂CO₃) zusammen mit einem Amin als Impfstoff verwendet. Der Impfstoff wird in geringen Dosen eingesetzt und erhöht katalytisch die Aufnahme von CO₂ durch das Karbonat. Vorzugsweise ist der Impfstoff Piperazin.

Vorzugsweise wird in der ersten Stufe die Flüssigkeit mit dem Gasgemisch im Gegenstrom geführt. Der Prozess ist also kontinuierlich, wobei aus dem Gaswäscher kontinuierlich Gasgemisch eingeleitet, vorgereinigtes Gas ausgeleitet und Flüssigkeit kontinuierlich ein- und ausgeleitet wird. Die ausgeleitete Flüssigkeit enthält hohe Anteile an chemisch absorbiertem Kohlendioxid, z.B. als Hydrogenkarbonat.

Zur Erhöhung der Aufnahme von Kohlendioxid durch die Flüssigkeit, bzw. durch die Substanz, wird die Oberfläche zwischen der gasförmigen und der flüssigen Phase möglichst erhöht. Hierzu kann beispielsweise die Flüssigkeit in der ersten Stufe zerstäubt werden, oder das Gasgemisch durch einen Tank mit der Flüssigkeit unter Bildung von Gasblasen geleitet werden, oder das Gasgemisch mit der Flüssigkeit über eine gasdurchlässige und flüssigkeitundurchlässige Membran kontaktiert werden.

Die erste Stufe erlaubt eine effiziente Bindung von Kohlendioxid in flüssiger Form - ohne das aufwändige Verflüssigungsverfahren unter Anwendung von hohen Drücken oder niedrigen Temperaturen zur CO₂-Verflüssigung angewendet werden müssen. Vorzugsweise wird die erste Stufe auch verwendet um in der zweiten Stufe abgetrenntes Kohlendioxid in flüssiger Phase zu binden. Hierzu kann das in der zweiten Stufe abgetrennte Kohlendioxid, insbesondere Kohlendioxidgas, der ersten Stufe zugeführt werden. Eine Kohlendioxid-Verflüssigung kann daher unterbleiben bzw. die erfindungsgemäße Vorrichtung hat vorzugsweise keinen Kohlendioxid-Verflüssiger - obwohl dieser natürlich in bestimmten Ausführungsformen sehr wohl vorgesehen werden kann. Die erfindungsgemäße Vorrichtung verfügt vorzugsweise über eine Gasleitung, welche abgetrenntes Kohlendioxid von der Kohlendioxidadsoptionsanlage oder der Molekularsiebanlage in den Gaswäscher führt.

Sofern eine Kohlendioxidverflüssigung abseits der ersten Stufe vorgenommen wird, kann dies kryogen erfolgen. Hierbei wird Kohlendioxid durch Abkühlen (kryogene Separation), gegebenenfalls auch unter Druck, vom Produktgas abgetrennt. Hierbei wird Kohlendioxid vorzugsweise auch verflüssigt, ein Vorgang, welcher die Abtrennung vom Produktgas erleichtert. Ein solcher Prozess ist beispielsweise in der EP 0646756 A1 oder der US 2004/0250682 A1 beschrieben und kann erfindungsgemäß herangezogen werden. Die kryogene Trennung beruht auf den unterschiedlichen Siedepunkten von Methan und Kohlendioxid. Üblicherweise wird das Biogas-Gasgemisch auf einen Überdruck von über 10 bar, vorzugsweise mindestens 12 bar, mindestens 14 bar, mindestens 16 bar, mindestens 17 bar, mindestens 18 bar, mindestens 19 bar, mindestens 20 bar oder höher verdichtet und auf eine Temperatur von kleiner gleich dem Siedepunkt von CO₂ bei diesen Druck, beispielsweise auf -40 °C, abgekühlt. Mittels eines Gasstrippers kann das in geringen Mengen im flüssigen Kohlendioxid gelöste Methan bei Bedarf abgetrennt werden, so dass hoch reines flüssiges CO₂ erhalten wird.

Die zweite Stufe ist vorzugsweise eine Membrantrennung. In der erfindungsgemäßen Vorrichtung ist vorzugsweise die Kohlendioxidadsoptionsanlage eine Druckwechseladsorptionsanlage, oder die Molekularsiebanlage eine Membrananlage für die Membrantrennung. Membranen, wie andere mögliche Molekularsiebe, erlauben eine gute Selektion von Kohlendioxid gegenüber Methan aus dem Gasgemisch. Die Membran wird zwischen zwei sich bildenden Abgasströmen vorgesehen, einem kohlendioxidabgereichteren Strom und einem kohlendioxidangereicherten Strom. Zur Trennung der Bereiche der beiden Ströme in der Anlage, kann diese z.B. in Form von Hohlfasern vorgesehen werden, durch die (oder um die) das vorgereinigte Gasgemisch geleitet wird. Der Abgasstrom aus dem Inneren der Hohlfasern bzw. der Abgasstrom um die Hohlfasern bilden die beiden getrennten Produktgase.

Ein Verfahren zur Membranseparation wird beispielsweise in der US 2009/156875 beschrieben und kann erfindungsgemäß eingesetzt werden. Geeignete Membranen zur Trennung von CO₂ und Methan sind im Patent US 5,674,629 beschrieben, z.B. PolyimidMembranen. Kurz, die Membrantrennung nutzt die unterschiedlichen Membranpermeationseigenschaften unterschiedlicher Gaskomponenten, auch von Methan und CO₂, um diese voneinander zu trennen. Bei Polyimidmembranen ist die Permeationsdifferenz von Methan und CO₂ relativ hoch, wobei CO₂ wesentlich schneller die Membran passiert. Bei gängigen Membranen gilt folgende Reihenfolge des Permeationsverhaltens von schnell zu langsam: H₂O, H₂, He, CO2, O₂, N₂, CH₄. Grundsätzlich ist es möglich, nur mit Membrantrennstufen Produktkonzentrationen von 99% Methan bzw. Kohlendioxid zu erzielen. Mit steigernder Konzentration im Reingas steigt bei diesem Verfahren jedoch der Energieaufwand für die Gasverdichtung und es ist eine mehrstufige Verfahrensführung notwendig.

Aufgrund der der erfindungsgemäßen Grobreinigung in der ersten Stufe ist es nicht notwendig diese zweite Stufe selber mehrstufige auszuführen. D.h. z.B. sind ein oder zweistufige Molekularsiebtrennungen (insbesondere Membrantrennungen) bzw. Adsoprtionstrennungen (z.B. PSA) ausreichend. In vorzugsweise Ausführungsformen der vorliegenden Erfindung wird daher eine Trennung in der zweiten Stufe (z.B. eine Membrantrennung) maximal ein oder zwei stufig durchgeführt.

Alternativ zur Membrantrennung kann auch eine Adsoption, z.B. durch eine PSA, vorgenommen werden. In der PSA wird diskontinuierlich Kohlendioxid (oder alternativ Methan) adsorbiert, wobei ein kohlendioxidabgreichertes Produktgas abgegeben wird und infolge Kohlendioxid (oder Methan) wieder desorbiert, wobei ein kohlendioxidreiches Gas - separat vom kohlendioxidabgreicherten Produktgas - abgegeben wird. Meist werden zwei Adsorber vorgesehen, wobei sich die Adsorption/Desorption zwischen den beiden Adsorbern wechselseitig abwechselt. Adsorber sind beispielsweise in der US 2011/005392 genannt. Die Adsorber können auch durch chemische Absorber ergänzt oder ersetzt werden, welche ebenfalls zur diskontinuierlichen Absorption oder Desorption geeignet sind. Vorzugsweise erfolgt der Wechsel zwischen Absorption und Desorption durch die Anlegung eines unterschiedlichen Druckes. Beispielsweise kann für die Absorption ein hoher Druck (Überdruck) und zur Desorption ein niedriger Druck (Unterdruck, Vakuum) eingestellt werden - z.B. durch Regelung am Produktgasausgang. Alternativ oder zusätzlich kann - je nach Adsorbermaterial - Adsorption und Desorption durch unterschiedliche Temperaturen reguliert werden.

Üblicherweise wird die zweite Stufe bei Überdrücken (Drücken größer als 1 bar) des eingeleiteten vorgereinigten Gasgemisches durchgeführt. Da die zweite Stufe nur ein- oder zweistufig ausgeführt werden muss, können geringere Drücke verwendet werden als bei üblichen Membrantrennverfahren. Vorzugsweise wird die zweite Stufe bei Gasdrücken des vorgereinigten Gasgemisches von bis zu 10 bar, vorzugsweise von bis zu 8 bar, insbesondere bevorzugt von bis zu 7 bar, im speziellen bevorzugt von zwischen 1,5 bar bis 6,5 bar, am meisten bevorzugt 2 bis 6 bar, durchgeführt. Vorzugsweise wird die Membrantrennung bei 4 bis 7 bar, im speziellen bei etwa 6 bar, durchgeführt. Eine PSA-Trennung wird vorzugsweise bei Drücken zwischen 1,5 bar und 4,5 bar, speziell bevorzugt bei etwa 3 bar, durchgeführt. Im Vergleich hierzu wird vorzugsweise die erste Stufe bei Gasdrücken von zwischen 0,8 bar bis 1,4 bar durchgeführt, die erste Stufe kann aber auch bei höheren Drücken durchgeführt werden. Diese Druckangaben verstehen sich als absolute Drücke und werden vorzugsweise durch Verdichtungen des Gasgemisches erzielt. Die Verdichtungsverhältnisse entsprechen den angegebenen Drücken, also zum Beispiel wird zur Erreichung eines Drucks von 3 bar eine Verdichtung von 1:3 vorgenommen. In der erfindungsgemäßen Vorrichtung kann hierzu ein Gasverdichter, zur Verdichtung des vorgereinigten Gasgemisches vor Einleitung in die Kohlendioxidadsoptionsanlage oder in die Membrananlage vorgesehen werden.

Das erfindungsgemäße zweistufige Verfahren zur Kohlendioxidabtrennung erlaubt hohe Reinheiten des Produktgases, indem beispielsweise nur 0,1 % bis 3 % Kohlendioxid verbleiben. Die erfindungsgemäße Kombination einer chemischen Grobreinigung (1. Stufe, Absorption) und einer meist physikalischen Feinreinigung (2. Stufe, Adsorption oder Siebtrennung) erlaubt die Erreichung dieses Ziels durch unerwartet geringen Energieaufwand. Vorzugsweise enthält das Gasgemisch Methan, welches bis zu einer Reinheit von mindestens 96 %, vorzugsweise mindestens 98%, durch die erfindungsgemäßen Verfahrensstufen gereinigt wird.

Vorzugsweise ist das Gasgemisch ein methanhaltiges Gas, vorzugsweise biogenen Ursprungs ("Biogas"), welches beispielsweise durch Fermentierung von Biomasse unter anaeroben Bedingungen erhalten werden kann. Vom Biomasse-Fermenter kann das Biogas über eine Leitung, und optional ein oder mehreren Vorreinigungsanlagen, Biogas in den Gaswäscher geleitet werden.

Biogas entsteht durch den natürlichen Prozess des mikrobiellen Abbaus organischer Stoffe unter anoxischen Bedingungen. Dabei setzen Mikroorganismen die enthaltenen Kohlenhydrate, Eiweiße und Fette in die Hauptprodukte Methan und Kohlenstoffdioxid um. Verfahren zur Fermentierung zur Bildung von Biogas sind unter anderem in der WO 03/06387 A2, EP 0646756 A1, WO 2009/137948 A2, DE 3243103 A1, beschrieben, und können erfindungsgemäß zur Fermentierung der Biomasse einsetzt werden.

Vor der Biogasaufbereitung besteht die wassergesättigte Gasmischung aus den Hauptkomponenten Methan (CH₄) und Kohlenstoffdioxid (CO2). In Spuren sind meist auch Stickstoff (N₂), Sauerstoff (O₂), Schwefelwasserstoff (H₂S), Carbonsulfid (COS), Wasserstoff (H₂) und Ammoniak (NH₃) enthalten. Für die Verwertung von Biogas ist der Methananteil am wichtigsten, da seine Verbrennung Energie freisetzt.

Weitere Gasreinigungen als Vor- oder Nachreinigung - vor oder nach der erfindungsgemäßen ersten oder zweiten Stufe - sind möglich. Beispielsweise wird in bestimmten Ausführungsformen das Gasgemisch vor Eintritt in die erste Stufe zur Entfernung von Schwefel oder Schwefelverbindungen und/oder Ammoniak und/oder Schwermetallen (welche bei der Fermentierung von verunreinigtem Biomüll im Gas auftreten können) vorgereinigt.

Derartige zusätzliche Reinigungsschritte sind beispielsweise die Ammoniakwäsche oder die Aktivkohlefiltrierung zur Entfernung von Schwefelverbindungen und Schwermetallen. Hierbei kann Ammoniak im Gasgemsich auf wenige ppm (z.B. 7 ppm) oder weniger reduziert werden.

Das in der (Wasch-)flüssigkeit des erfindungsgemäßen ersten Schrittes angereicherte Kohlendioxid kann (optional) transportiert und wieder desorbiert werden, z.B. - je nach Flüssigkeit bzw. absorbierenden Substanzen darin - chemisch oder vorzugsweise durch Temperaturerhöhung (z.B bei Hydrogencarbonaten). Vorzugsweise wird das gewonnene Kohlendioxid zur Verwertung in Glas-, Gewächs- oder Treibhäusern verwendet, wo es durch Pflanzen aufgenommen wird. Dies ist eine echte CO₂-Senke ohne die Umwelt zu belasten.

Das erhaltene Methan nach der ersten oder nach der zweiten Stufe oder ein Teil des ursprünglichen Gasgemisches kann zum Betrieb eines Heizkraftwerks benutzt werden. Im Heizkraftwerk wird vorzugsweise durch eine Gasturbine elektrische Energie und Wärme erzeugt. Durch Verbrennung erzeugte Wärme, insbesondere der Abwärme des Kraftwerks, wird vorzugsweise zur Desorption von Kohlendioxid von der Flüssigkeit bzw. den absorbierenden Substanzen verwendet. Dadurch wird die Flüssigkeit regeneriert und kann im erfindungsgemäßen Verfahren wieder genützt werden. Der Großteil des gereinigten Methans wird in das Erdgasnetz eingespeist. Somit lassen sich durch das erfindungsgemäße Verfahren sehr viele Synergien nutzen, wodurch eine hohe Effizienz erreicht wird, wobei zusätzlich wirtschaftlich wertvoll Produkte abgeleitet werden können.

Die vorliegende Erfindung wird weiters durch die folgenden

Figuren und Beispiele erläutert ohne auf diese speziellen Ausführungsformen der Erfindung limitiert zu sein.

In den Figuren zeigt
Fig. 1 schematisch eine Vorrichtung zur Gasreinigung und der Aufkonzentration des Produktgases mit externer Wärmequelle;
Fig. 2 schematisch eine Vorrichtung zur Gasreinigung und der Aufkonzentration des Produktgases mit CO₂ Verflüssigung des CO₂ aus dem Gaswäscher und dem Membranverfahren
Fig. 3 schematisch eine Vorrichtung zur Gasreinigung und der Aufkonzentration des Produktgases mit der Verwertung des CO₂ aus dem Gaswäscher in einem Glashaus
Fig. 4 eine Darstellung der Komponenten einer Gasturbine
Fig. 5 schematisch eine Vorrichtung zur Gasreinigung und der Aufkonzentration des Produktgases mit einer Verflüssigung des CO₂ zur Erzeugung eines CO₂-Gases mit sehr geringem Methanschlupf unter Einbindung einer Gasturbine zur Wärmeerzeugung und zur Erzeugung elektrischer Leistung.
Fig. 6 eine vereinfachte Darstellung eines BHKW (Bioheizkraftwerk)
Fig. 7 schematisch eine Vorrichtung zur Gasreinigung und der Aufkonzentration des Produktgases mit einer Verflüssigung des CO₂ zur Erzeugung eines CO₂-Gases mit sehr geringem Methanschlupf unter Einbindung eines BHKW zur Wärmeerzeugung und zur Erzeugung elektrischer Leistung.
Fig. 8 schematisch eine Vorrichtung zur Gasreinigung und der Aufkonzentration von Methan mit einem Waschverfahren
Fig. 9 schematisch eine Vorrichtung zur Gasreinigung und der Aufkonzentration des Produktgases mit einer Verflüssigung des CO₂

In den Figuren sind identische bzw. einander entsprechende Bereiche, Bauteile, Bauteilgruppen oder Verfahrensschritte mit denselben Bezugszeichen gekennzeichnet. Die Flussrichtungen in Leitungen sind mit Pfeilen gekennzeichnet.

### Beispiele

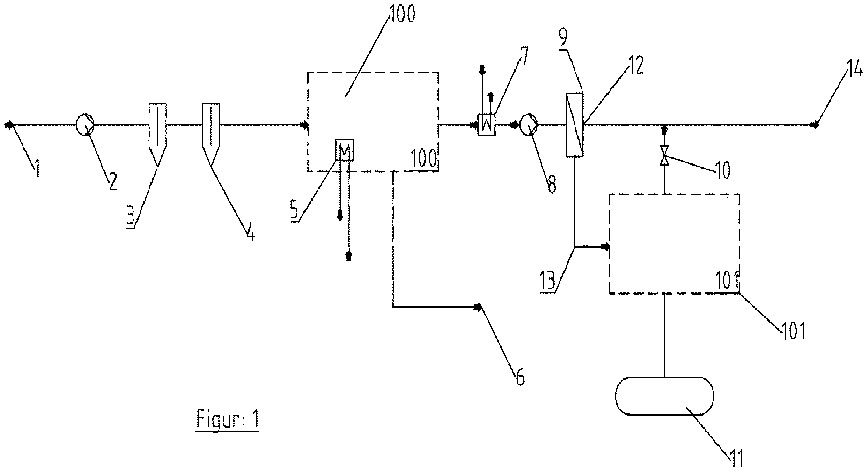

Fig. 1 zeigt das erfindungsgemäße Verfahren, wobei über die Rohrleitung 1 das Rohbiogas eingebracht wird. Mit dem Vorverdichter 2 wird das Rohbiogas verdichtet und dem Ammoniakwäscher 3 zugeleitet, danach erfolgt die Zuleitung zum Aktivkohlefilter 4. Der erfindungsgemäße Gaswäscher ist in der Box 100 dargestellt. Die externe Wärmezuführung erfolgt über den Wärmetauscher 5, das aus dem Waschverfahren abgetrennte CO₂ (Kohlendioxid) wird über die Leitung 6 abgeleitet. Das an Methan aufkonzentrierte Biogas wird über den Wärmetauscher 7 abgekühlt und so das im Gas enthaltene Wasser durch Unterschreitung des Taupunktes ausgeschieden. Danach erfolgt die Hochverdichtung 8 um dann das Biogas als Feed der Membran 9 zuzuführen. Das Permeat ist aufkonzentriertes Biomethan, das zusammen mit dem Restmethan 10 das Biomethan 14 ergibt. Das im Membranverfahren abgetrennte CO₂ 13 wird einer CO₂-Verflüssigung zugeführt 101 aus der das flüssige CO₂ im Tank 11 gelagert wird.

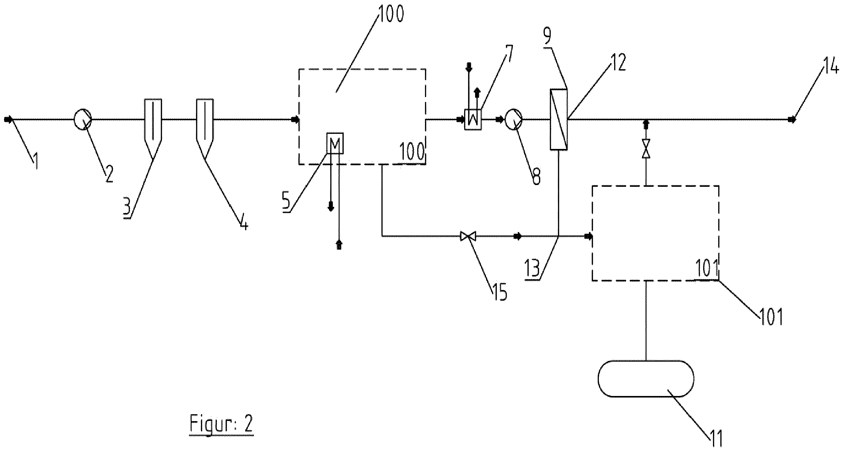

Fig. 2 zeigt das erfindungsgemäße Verfahren der Erzeugung von Biomethan wie in Figur 1 beschrieben. Das aus dem Gaswäscher abgetrennte CO₂ wird über die Drosselarmatur der CO₂-Verflüssigung zugeführt und das flüssige CO₂ im Tank 11 gelagert.

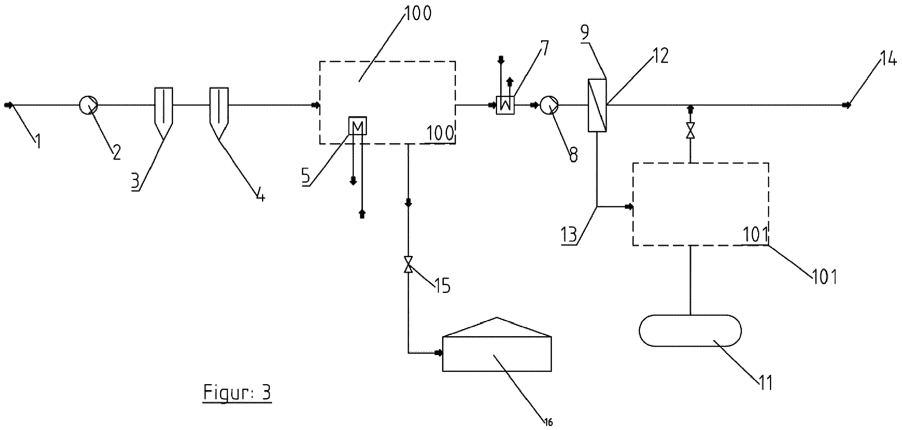

Fig. 3 zeigt das erfindungsgemäße Verfahren der Erzeugung von Biomethan wie in Figur 1 beschrieben. Das aus dem Gaswäscher abgetrennte CO₂ wird über die Drosselarmatur einem Glashaus oder Gewächshaus, Pflanzenhaus zugeführt und dort in den Pflanzen zu Zucker, Stärke und Sauerstoff verarbeitet.

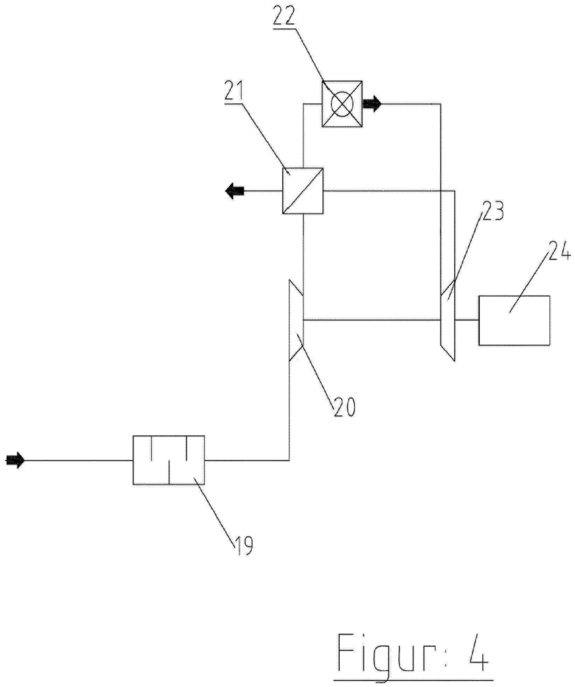

Fig. 4 zeigt eine Gasturbine bestehend aus einem Luft Einlass und Schalldämpfer 19, einem Verdichter 20, der über eine Welle mit der Turbine 23 verbunden ist, die ihrerseits wieder mit einem Generator 24 verbunden ist. Die verdichtete Verbrennungsluft wird über den Regenerator 21 erwärmt, und der Brennkammer 22 zugeführt. Das heiße Abgas wird über die Turbine entspannt und das Abgas dem Regenerator zugeführt.

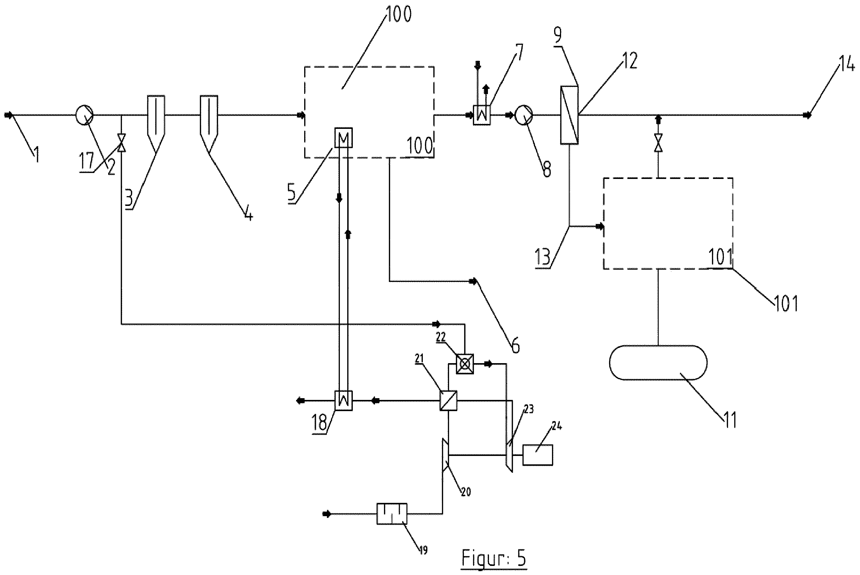

Fig. 5 zeigt die erfindungsgemäße Einbindung der Gasturbine in das Verfahren, wobei über die Armatur 17 ein entsprechender Anteil an Rohbiogas der Gasturbinenbrennkammer 22 zugeführt wird. Die Abwärme des Abgases aus dem Regenerator 22 wird nun über den Wärmetauscher 18 dem Heißwasserkreislauf zugesendet, der über den Wärmetauscher 5, die notwendige Wärme für den Desorber in der Gaswäsche zur Verfügung stellt. Die restliche Abwärme im Abgas weiter verströmt werden. Über den Generator 24 wird elektrische Leistung für den Eigenbedarf zur Einspeisung ins lokale E-Netz erzeugt.

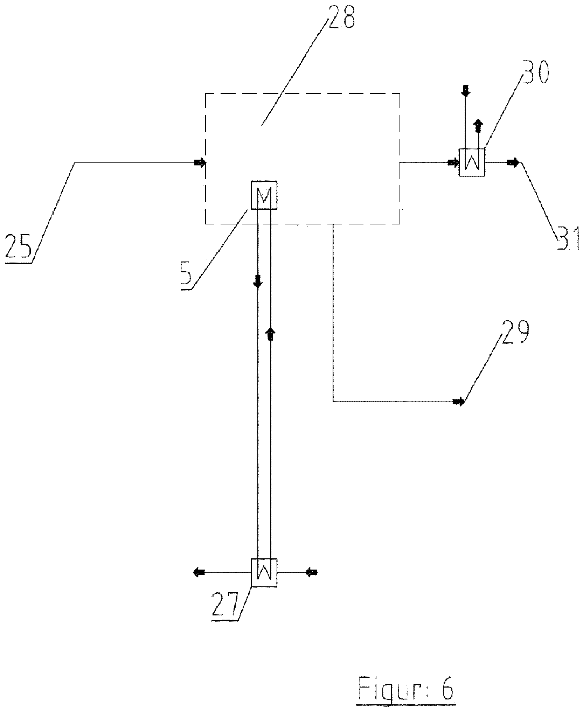

Fig. 6 zeigt das vereinfachte Schema eines BHKW mit der Zuführung von Rohbiogas 25 dem BHKW (Bioheizkraftwerk) 28 und dem Abgas 31. In der Abgasleitung ist ein Abwärmetauscher 30 enthalten. Die Niedertemperaturwärme aus dem Öl und Kühlwasserkreislauf wird im Wärmetauscher 27 verwertet.

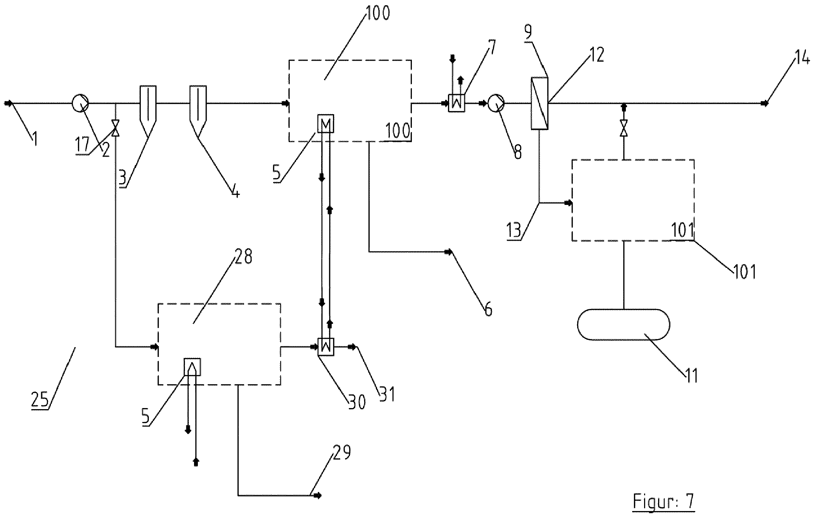

Fig. 7 zeigt die erfindungsgemäße Einbindung eines BHKW 28 zur Erzeugung von elektrischer Energie und Wärme. Die Abwärme 30 aus dem Abgas wird für den Waschprozess 100 verwendet. Das Rohbiogas 25 wird über die Armatur 17 dem BHKW zugeführt. Das abgekühlte Abgas 31 wird an die Umgebung abgegeben.

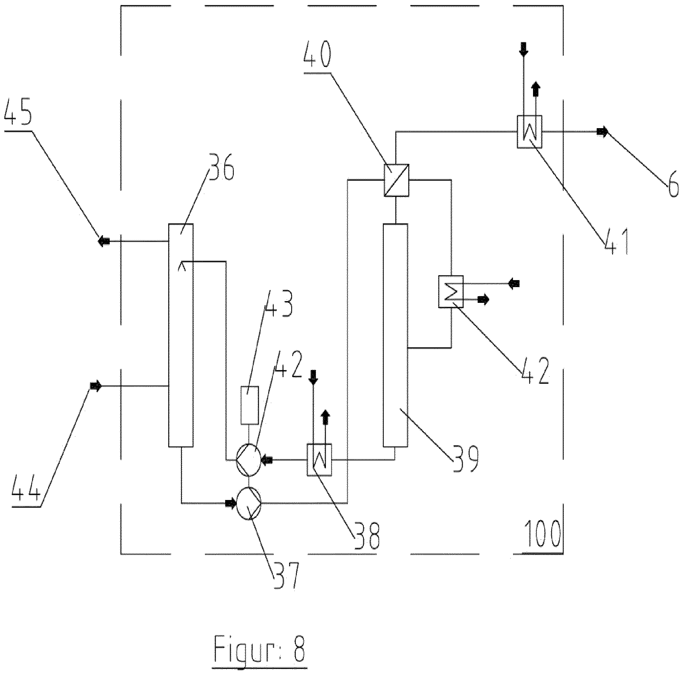

Fig. 8 zeigt die erfindungsgemäße Einbindung eines Waschprozesses mit der Zuführung des Rohbiogases 44 und der Ableitung des Rohbiogases 45 aus dem Absorber. Der Absorber 36 wird mit dem Waschmittel beladen, das beim Durchströmen das CO₂ aus dem Rohbiogas absorbiert. Die Pumpturbine 37,42,43 bestehend aus der Pumpe 42, der Turbine 42 und des Motors 43 verdichtet das Waschmittel auf einen Desorptionsdruck. Das Waschmittel wird einem Regenerator 40 zugeführt und dann über einen Wärmetauscher 42 dem Desorber 39 zugeführt wo die Regeneration des Waschmittels erfolgt. Das abgetrennte CO₂ 6 wird über den Wärmetauscher 41 abgekühlt.

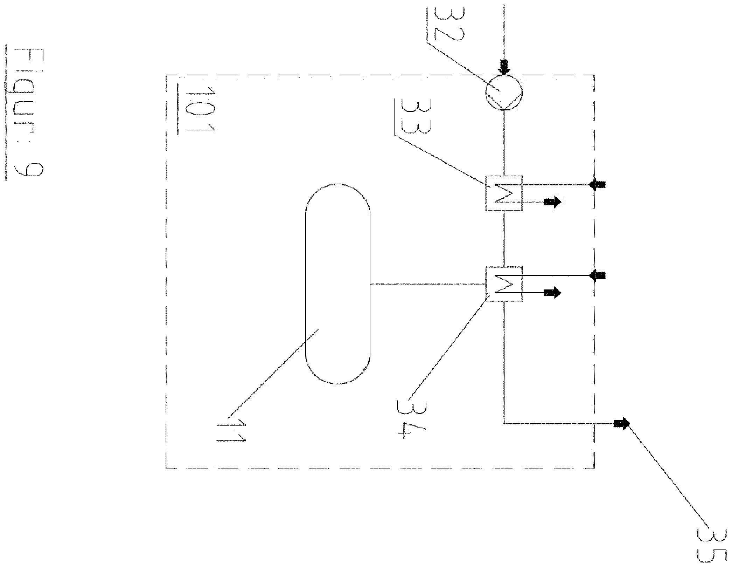

Fig. 9 zeigt die erfindungsgemäße Einbindung eines Verflüssigungsverfahrens, wo das dampfförmige CO₂ über einen Verdichter 32 auf einen Druck von 18 bar gebracht wird, das so verdichtete CO₂ Gas abgekühlt wird, und über den Wärmetauscher 34 auf eine Temperatur von -25°C abgekühlt wird. Das flüssige CO₂ wird im Tank 11 gespeichert, das gasförmige CH₄ wird über die Leitung 35 abgeleitet.

## Patentansprüche

1. Verfahren zur Entfernung von Kohlendioxid aus einem Kohlendioxid-haltigen Gasgemisch, umfassend eine mehrstufige Gasreinigung, **dadurch gekennzeichnet, dass** in zumindest einer ersten Stufe das Gasgemisch mit einer Kohlendioxid-absorbierenden Flüssigkeit kontaktiert wird, wodurch ein vorgereinigtes Gasgemisch erhalten wird, und in zumindest einer zweiten Stufe das vorgereinigte Gasgemisch mit einem Kohlendioxid-Adsorber oder einem Molekularsieb kontaktiert wird, wodurch im vorgereinigten Gasgemisch verbliebenes Kohlendioxid abgetrennt wird.

2. Verfahren nach Anspruch 1, **dadurch gekennzeichnet, dass** die zweite Stufe eine Membrantrennung umfasst.

3. Verfahren nach Anspruch 2, **dadurch gekennzeichnet, dass** die eine Membrantrennung maximal ein oder zwei stufig durchgeführt wird.

4. Verfahren nach einem der Ansprüche 1 bis 3, **dadurch gekennzeichnet, dass** die zweite Stufe bei Gasdrücken des vorgereinigten Gasgemisches von bis zu 10 bar, vorzugsweise von bis zu 8 bar, insbesondere bevorzugt von bis zu 7 bar, im speziellen bevorzugt von zwischen 1,5 bar bis 6,5 bar, durchgeführt wird.

5. Verfahren nach einem der Ansprüche 1 bis 4, **dadurch gekennzeichnet, dass** die erste Stufe bei Gasdrücken von zwischen 0,8 bar bis 1,4 bar durchgeführt wird.

6. Verfahren nach einem der Ansprüche 1 bis 5, **dadurch gekennzeichnet, dass** in der ersten Stufe die Flüssigkeit mit dem Gasgemisch im Gegenstrom geführt wird.

7. Verfahren nach einem der Ansprüche 1 bis 6, **dadurch gekennzeichnet, dass** die Flüssigkeit in der ersten Stufe zerstäubt wird, oder dass das Gasgemisch durch einen Tank mit der Flüssigkeit unter Bildung von Gasblasen geleitet wird, oder dass das Gasgemisch mit der Flüssigkeit über eine gasdurchlässige und flüssigkeitundurchlässige Membran kontaktiert wird.

8. Verfahren nach einem der Ansprüche 1 bis 7, **dadurch gekennzeichnet, dass** das in der zweiten Stufe abgetrennte Kohlendioxid, insbesondere Kohlendioxidgas, der ersten Stufe zugeführt wird.

9. Verfahren nach einem der Ansprüche 1 bis 8, **dadurch gekennzeichnet, dass** das Gasgemisch vor Eintritt in die erste Stufe zur Entfernung von Schwefel oder Schwefelverbindungen und/oder Ammoniak und/oder Schwermetallen vorgereinigt wird.

10. Verfahren nach einem der Ansprüche 1 bis 9, **dadurch gekennzeichnet, dass** die Kohlendioxid-absorbierende Flüssigkeit eine Kohlendioxid-absorbierende Substanz enthält, vorzugsweise ausgewählt aus einem wasserlöslichem Carbonat-, Hydrogencarbonat oder Bicarbonatbilder, oder einer Base oder einem Amin.

11. Vorrichtung geeignet zur Durchführung eines Verfahrens der Ansprüche 1 bis 10 mit einem Gaswäscher mit einer Einleitung für ein Gasgemisch, Ein- und Ausgangsleitungen für eine Waschflüssigkeit, einer Ausgangsleitung für ein vorgereinigtes Gasgemisch, und mit einer Kohlendioxidadsoptionsanlage oder einer Molekularsiebanlage zur Abtrennung von Kohlendioxid aus dem vorgereinigten Gasgemisch.

12. Vorrichtung nach Anspruch 11, **dadurch gekennzeichnet, dass** die Kohlendioxidadsoptionsanlage eine Druckwechseladsorptionsanlage ist, oder die Molekularsiebanlage eine Membrananlage ist.

13. Vorrichtung nach Anspruch 11 oder 12 mit einem Gasverdichter, zur Verdichtung des vorgereinigten Gasgemisches vor Einleitung in die Kohlendioxidadsoptionsanlage oder in die Membrananlage.

14. Vorrichtung nach einem der Ansprüche 11 bis 13 mit einer Gasleitung, welche abgetrenntes Kohlendioxid von der Kohlendioxidadsoptionsanlage oder der Molekularsiebanlage in den Gaswäscher führt.

15. Vorrichtung nach einem der Ansprüche 11 bis 14 mit einem Biomasse-Fermenter, von dem über eine Leitung, und optional ein oder mehreren Vorreinigungsanlagen, Biogas in den Gaswäscher geleitet wird.
